(19) Europäisches Patentamt / European Patent Office / Office européen des brevets

(11) **EP 1 926 701 B1**

(12) # EUROPÄISCHE PATENTSCHRIFT

(45) Veröffentlichungstag und Bekanntmachung des Hinweises auf die Patenterteilung:
**12.03.2014 Patentblatt 2014/11**

(21) Anmeldenummer: **06792911.7**

(22) Anmeldetag: **21.08.2006**

(51) Int Cl.:
***C07C 209/02*** (2006.01) ***C07C 211/46*** (2006.01)
***B01D 69/02*** (2006.01) ***B01D 71/02*** (2006.01)
***C01B 3/50*** (2006.01) ***B01D 53/22*** (2006.01)
***B01D 67/00*** (2006.01) ***B01D 69/00*** (2006.01)
***B01D 69/10*** (2006.01)

(86) Internationale Anmeldenummer:
**PCT/EP2006/065474**

(87) Internationale Veröffentlichungsnummer:
**WO 2007/025882 (08.03.2007 Gazette 2007/10)**

(54) **Direktaminierung von Benzol**

Direct amination of benzene

Amination directe de benzene

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **30.08.2005 DE 102005041140**

(43) Veröffentlichungstag der Anmeldung:
**04.06.2008 Patentblatt 2008/23**

(73) Patentinhaber: **BASF SE**
**67056 Ludwigshafen (DE)**

(72) Erfinder:
- **VAN LAAR, Frederik**
  **Dubai (AE)**
- **SCHWAB, Ekkehard**
  **67434 Neustadt (DE)**
- **VOSS, Hartwig**
  **67227 Frankenthal (DE)**
- **ANDERS, Joachim-Thierry**
  **67161 Gönnheim (DE)**
- **CRONE, Sven**
  **67117 Limburgerhof (DE)**
- **MACKENROTH, Wolfgang**
  **67089 Bad Dürkheim (DE)**

(56) Entgegenhaltungen:
**WO-A-00/09473 WO-A-00/32512**
**WO-A1-00/69804 GB-A- 2 078 539**

- **A.S. DARLING ET AL: "Trennung und Reinigung von Wasserstoff durch Permeation an Membranen aus Palladium-Legierungen" CHEMIE-ING.-TECHN., Bd. 1, Nr. 37, 1965, Seiten 18-27, XP002413380 in der Anmeldung erwähnt**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 926 701 B1

Printed by Jouve, 75001 PARIS (FR)

## Beschreibung

Die Erfindung betrifft ein Verfahren zur Direktaminierung von Benzol durch Umsetzung von Benzol mit Ammoniak, wobei man Wasserstoff aus dem Reaktionsgemisch physikalisch entfernt. Insbesondere bezieht sich die Erfindung auf Verfahren zur Aminierung von Benzol durch Umsetzung von Benzol mit Ammoniak insbesondere gemäß der folgenden Reaktion, die bevorzugt katalysiert ist:

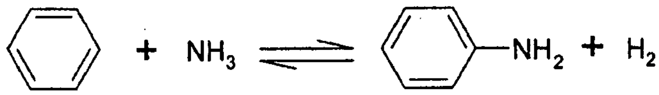

wobei man Wasserstoff, insbesondere den bei der Aminierung entstehenden Wasserstoff, durch eine Wasserstoff-permeable Membran aus dem Reaktionsgemisch entfernt.

Die kommerzielle Herstellung von Aminen, insbesondere von aromatischen Aminen, wie Anilin, wird üblicherweise in mehrstufigen Reaktionen durchgeführt. Anilin wird beispielsweise üblicherweise durch Umwandlung von Benzol in ein Benzol-Derivat, z.B. Nitrobenzol, Chlorbenzol oder Phenol und anschließende Umwandlung dieses Derivats in Anilin hergestellt.

Vorteilhafter als solche indirekten Verfahren zur Herstellung von insbesondere aromatischen Aminen sind Methoden, die eine direkte Herstellung der Amine aus den entsprechenden Kohlenwasserstoffen ermöglichen. Eine sehr elegante Route ist die heterogenkatalysierte Direktaminierung von Benzol, erstmals 1917 von Wibaut (Berichte, 50, 541-546) beschrieben. Da die Direktaminierung gleichgewichtslimitiert ist, wurden mehrere Systeme beschrieben, welche die Gleichgewichtslimitierung durch die selektive Entfernung von Wasserstoff aus der Reaktion verschiebt und einen erhöhten Benzolumsatz ermöglicht. Die meisten Verfahren basieren auf dem Einsatz von Metalloxiden, welche durch Wasserstoff reduziert werden, damit den Wasserstoff aus dem Reaktionssystem entfernen und somit das Gleichgewicht verschieben.

CN 1555921 A offenbart die Oxido-aminierung von Benzol in der Flüssigphase, wobei als "O"-Donor Wasserstoffperoxyd fungiert. Die Anwendung von $H_2O_2$ ist allerdings aufgrund des Preises und der geringen Selektivität aufgrund von Folgereaktionen für Massenchemikalien nur bedingt geeignet.

In CA 553,988 ist ein Verfahren zur Herstellung von Anilin aus Benzol offenbart, worin Benzol, Ammoniak und gasförmiger Sauerstoff bei einer Temperatur von etwa 1000°C an einem Platinkatalysator umgesetzt werden. Geeignete Platin enthaltende Katalysatoren sind Platin allein, Platin mit bestimmten spezifischen Metallen und Platin zusammen mit bestimmten spezifischen Metalloxiden. Des weiteren ist in CA 553,988 ein Verfahren zur Herstellung von Anilin offenbart, worin Benzol in der Gasphase mit Ammoniak in Anwesenheit eines reduzierbaren Metalloxids bei Temperaturen von 100 bis 1000°C umgesetzt wird, ohne Zugabe von gasförmigem Sauerstoff. Geeignete reduzierbare Metalloxide sind die Oxide des Eisens, Nickels, Kobalts, Zinns, Antimons, Bismuts und Kupfers.

US 3,919,155 betrifft die Direktaminierung von aromatischen Kohlenwasserstoffen mit Ammoniak, wobei als Katalysator Nickel/Nickeloxid eingesetzt wird, wobei der Katalysator zusätzlich Oxide und Carbonate von Zirkonium, Strontium, Barium, Calcium, Magnesium, Zink, Eisen, Titan, Aluminium, Silizium, Cer, Thorium, Uran und Alkalimetallen enthalten kann.

US 3,929,889 bezieht sich ebenfalls auf die Direktaminierung von aromatischen Kohlenwasserstoffen mit Ammoniak an einem Nickel/Nickeloxidkatalysator, wobei der eingesetzte Katalysator teilweise zu elementarem Nickel reduziert wurde und anschließend reoxidiert wurde um einen Katalysator zu erhalten, der ein Verhältnis von Nickel: Nickeloxid von 0,001 : 1 bis 10 : 1 aufweist.

US 4,001,260 offenbart ein Verfahren zur Direktaminierung von aromatischen Kohlenwasserstoffen mit Ammoniak, wobei wiederum ein Nickel/Nickeloxidkatalysator eingesetzt wird, der auf Zirkoniumdioxid aufgebracht ist, und vor Einsatz in der Aminierungsreaktion mit Ammoniak reduziert wurde.

US 4,031,106 betrifft wiederum die Direktaminierung von aromatischen Kohlenwasserstoffen mit Ammoniak an einem Nickel/Nickeloxidkatalysator auf einem Zirkoniumdioxidträger, der weiterhin ein Oxid, ausgewählt aus Lanthanoiden und Seltenerdmetallen, enthält.

DE 196 34 110 beschreibt die nicht oxidative Aminierung bei einem Druck von 10-500 bar und einer Temperatur von 50-900°C, wobei die Umsetzung in Gegenwart eines sauren Heterogenkatalysators erfolgt, der mit leichten und schweren Platinmetallen modifiziert ist.

WO 00/09473 beschreibt ein Verfahren zur Herstellung von Aminen durch Direktaminierung von aromatischen Kohlenwasserstoffen an einem Katalysator, enthaltend mindestens ein Vanadiumoxid.

WO 99/10311 lehrt ein Verfahren zur Direktaminierung von aromatischen Kohienwasserstoffen bei einer Temperatur von < 500°C und einem Druck von < 10 bar. Als Katalysator wird ein Katalysator enthaltend mindestens ein Metall ausgewählt aus Übergangsmetallen, Lanthaniden und Actiniden, bevorzugt Cu, Pt, V, Rh und Pd, eingesetzt.

Bevorzugt wird die Direktaminierung zur Erhöhung der Selektivität und/oder des Umsatzes in Anwesenheit eines Oxidationsmittels durchgeführt.

WO 00/69804 betrifft ein Verfahren zur Direktaminierung von aromatischen Kohlenwasserstoffen, wobei als Katalysator ein Komplex eingesetzt wird, enthaltend ein Edelmetall und ein reduzierbares Metalloxid. Dabei sind Katalysatoren, enthaltend Palladium und Nickeloxid bzw. Palladium und Kobaltoxid, besonders bevorzugt.

Alle genannten Verfahren gehen dabei von einem Mechanismus zur Direktaminierung aus, wie er in der Zusammenfassung von WO 00/69804 aufgeführt ist. Danach erfolgt zunächst die (edel)metallkatalysierte Herstellung der gewünschten Aminverbindung aus dem aromatischen Kohlenwasserstoff Und Ammoniak und in einem zweiten Schritt das "Abfangen" des im ersten Schritt entstandenen Wasserstoffs mit einem reduzierbaren Metalloxid. Die gleichen mechanistischen Überlegungen werden dem Verfahren in WO 00/09473 zugrundegelegt, worin der Wasserstoff mit Sauerstoff aus Vanadiumoxiden abgefangen wird (Seite 1, Zeilen 30 bis 33). Der gleiche Mechanismus wird auch in US 4,001,260 zugrundegelegt, wie aus den Ausführungen und der Abbildung in Spalte 2, Zeilen 16 bis 44 ersichtlich ist.

WO 00/32512 bezieht sich auf die Verwendung einer wasserstoffselektiven Membran zur Abtrennung und Aufreinigung von Wasserstoff bei einer diskontinuierlich durchgeführten Dehydrierungsreaktion oder Wasserstoff erzeugenden Reaktion.

Aufgabe der vorliegenden Erfindung ist es, ein besonders wirtschaftliches Verfahren zur Umsetzung von Benzol mit Ammoniak zu entwickeln, bei dem ein kontinuierliches Verfahren bei möglichst hoher Selektivität ermöglicht wird.

Diese Aufgabe wird dadurch gelöst, dass man Wasserstoff aus dem Reaktionsgemisch gemaß Anspruch 1 physikalisch entfernt.

Es würde überraschenderweise gefunden, dass der Umsatz bei der Direktaminierung an Metallkatalysatoren (zum Beispiel Ni, Fe, Co, Cu, EM oder deren Legierungen, wobei EM für Edelmetalle steht), verglichen mit dem Gleichgewichtsumsatz, erheblich gesteigert werden könnte, wenn man den bei der Umsetzung des Kohlenwasserstoffs mit dem Ammoniak entstehenden Wasserstoff durch Einsatz von wasserstoffpermeablen, bevorzugt Wasserstoff-selektiven Membranen aus dem Reaktionsgemisch entfernt. Solche Membranen als auch deren Herstellverfahren sind aus der Literatur bekannt. Durch Anwendung dieses Verfahrens kann über längere Perioden ohne Desaktivierung Anilin produziert werden. Im Vergleich zur Zugabe von einem sauerstoffhaltigen Gas muss keine aufwendige Gastrennung nach Reaktion durchgeführt werden.

Bei den eingangs beschriebenen, bekannten metall-metalloxidischen Systemen muss der Katalo-reaktant nach einiger Zeit wieder mit "Sauerstoff" aufgeladen worden. Dies bedeutet kostenintensive Unterbrechungen, da die Aminierung und die Reaktivierung üblicherweise nicht bei den gleichen Bedingungen (Druck und Temperatur) ablaufen. Der Reaktor muss somit üblicherweise entspannt, gespült und inertisiert, der Katalysator reaktiviert, und wieder unter Reaktionsbedingungen gebracht werden. Die Selektivität der gesamten Reaktion ändert sich mit dem Säuerstoffinhalt des Kataloreaktants und muss also durch Verfahrensänderung (Druck, Ammoniak-Aromaten-Verhältnis und/oder Temperatur) kompensiert werden. Eine hinreichende Selektivität für die C als auch N Bilanz kann mit diesen Systemen nicht erreicht werden, da Ammoniak durch die Metalloxide oder durch adsorbierten Sauerstoff an der Oberfläche unter Bildung von $N_2$ und $H_2O$ verbrennt wird. Bei der Zugabe von höheren Sauerttoffkonzentrationen kann Ammoniak nicht nur in Wasser und Stickstoff umgewandelt werden, sondern auch zu NOx. Auch die eingesetzten Kohlenwasserstoffe, insbesondere die Aromaten können durch eine zu hohe Sauerstoffkonzentration verbrannt werden. Durch Verdünnung kann die Sauerstoffkonzentration gesteuert werden. Dies bedeutet jedoch auch, dass die Verdünnungskomponente (üblicherweise $N_2$ bei Luftzugabe) aufwendig aus dem Reaktionsgemisch entfernt werden muss. Die Bereitstellung einer vollintegrierten Lösung ist somit mit metalloxidischen Systemen schwierig oder gar nicht zu bewerkstelligen.

Diese Nachteile werden durch die erfindungsgemäße physikalische Entfernung des Wasserstoffs aus dem Reaktionssystem vermieden. Das erfindungsgemäße Verfahren ermöglicht somit eine sehr effiziente, selektive, kostengünstige Direktaminierung.

Unter dem Ausdruck "physikalisch entfernen" ist zu verstehen, dass der Wasserstoff physisch und bevorzugt selektiv aus dem Reaktionsgemisch entweicht. Im Vergleich zu bekannten Verfahren, bei denen die Konzentration von Wasserstoff in dem Reaktionsgemisch durch Reaktion erniedrigt wird, kann erfindungsgemäß auf die Folgereaktion und insbesondere die Zugabe von gegenüber Wasserstoff reaktiven Komponenten zum Reaktionssystem verzichtet werden. Auch muss keine Abtrennung dieser im Grunde unerwünschten Folgeprodukte vom eigentlichen Verfahrensprodukt, bevorzugt dem Anilin, erfolgen.

Die physikalische Entfernung des Wasserstoffs aus dem Reaktionsgemisch erfolgt, in dem man Wasserstoff durch eine Wasserstoff- permeable, bevorzugt Wasserstoff-selektive Membran aus dem Reaktionsgemisch entfernt, bevorzugt indem der Wasserstoff aus dem Reaktionsgemisch durch die Membran diffundiert. Die Diffusion des Wasserstoffs ist dabei bevorzugt getrieben durch das Konzentrationsgefälle zwischen dem Reaktionssystem (Retentatseite), in dem durch die Reaktion von Benzol mit Ammoniak Wasserstoff entsteht, und dem auf der anderen Seite der Membran befindlichen Raum (Permeatseite). Der auf die Permeatseite diffundierte Wasser-stoff wird dort bevorzugt durch Abtransport, beispielsweise mittels Gasstrom oder Unterdruck, und/oder durch chemische Reaktion, beispielsweise durch Reduktion einer organische Verbindung in der Gasphase, zum Beispiel Benzol zu Cyclohexan oder unter Bildung von

Wasser, bevorzugt mit einem sauerstoffhaltigen Gas, wie zum Beispiel Luft, bevorzugt durch katalysierte Reaktion mit Sauerstoff und/oder Luft, abgereichert, d.h. entfernt. Dadurch wird das Konzentrationsgefälle zwischen Retentatseite und Permeatseite, das die Diffusion treibt, aufrechterhalten oder erhöht.

Die Wasserstoff-permeable Membran ist Teil eines Reaktors und kann beispielsweise den Reaktionsraum, in dem Benzol mit Ammoniak umgesetzt wird, zumindest teilweise begrenzen. Das erfindungsgemäße Verfahren erfolgt derart, dass die Umsetzung von Benzol mit Ammoniak in einem Membranreaktor mit integrierter Wasserstoffabtrennung durch eine Wasserstoff- permeable Membran erfolgt.

Bevorzugt weist die Membran eine Permeanz für Wasserstoff von größer 10 $m^3 /(m^2 \times h \times bar^n)$, besonders bevorzugt > 50 $m^3 /(m^2 \times h \times bar^n)$ auf, wobei theoretisch n=0,5 und real zwischen 0,5 und 0,6 bedeutet, somit bevorzugt n zwischen 0,5 und 0,6, besonders bevorzugt n = 0,5. Die Permeanz (P) kann aus dem Wasserstofffluss (in $m^3/(m^2,h)$ und den Wasserstoffpartialdrücken berechnet werden:

$$P = \frac{Wasserstofffluss}{P^n_{Retentat} - P^n_{Permeat}}$$

Die Membran ist bevorzugt möglichst selektiv für Wasserstoff. D.h., dass die Membran bevorzugt dicht ist und besonders bevorzugt eine $H_2$ /$N_2$ Selektivität > 1000 aufweist. Durch Einsatz solcher Membranen wird gewährleistet, dass nur ein möglichst geringer Anteil an Edukt (Benzol, Ammoniak) und/oder Produkt (Anilin) auf die Permeatseite der Membran gelangt. Für die bevorzugten Pd und Pd-Legierte Membrane können die Edukte und Kohlenwasserstoffe nicht durch die Membran diffundieren.

Die Membran weist bevorzugt eine Dicke zwischen 0,1 $\mu$m und 25 $\mu$m, besonders bevorzugt zwischen 0,5 $\mu$m und 10 $\mu$m, ganz besonders bevorzugt zwischen 1$\mu$m und 5 $\mu$m auf.

Die Membran kann selbsttragend aufgebaut sein. Aufgrund der im Regelfall sehr hohen Materialkosten kann es von Vorteil sein, die eigentliche Membran auf einer porösen keramischen und/oder metallischen Stützschicht zu fixieren ("Composit"-Membran). Dies bietet zudem den Vorteil, dass die Membran stabilisiert wird und außerdem geringe Schichtdicken ermöglicht werden. Typische Membranen können zum Beispiel bei NGK in Japan oder bei Johnson Matthey gekauft werden.

Als geeignete Membranen kommen beispielsweise mesoporöse anorganische Membranen, mikroporöse anorganische Membranen, Polymer-Membranen, Membranen auf Basis von Metallen der 4. oder 5. Nebengruppe beschichtet mit Palladium, nanokristalline Metallmembranen, gemischtleitende Membranen und bevorzugt Membranen auf der Basis von Palladium oder Palladiumlegierungen in Frage.

Mesoporöse anorganische Membranen sind beispielsweise solche mit einer Porengröße kleiner 50 nm, z.B. solche auf Basis von $Al_2O_3$.

Mikroporöse anorganische Membranen sind beispielsweise solche mit einer Porengröße kleiner 2 nm, z.B. solche auf Basis von keramischen- oder Kohlenstoff-Molekularsieben. Als keramische Molekularsieb-Membranen kommen zeolithische, beispielsweise solche vom MFI-Typ (ZSM-5 oder Silicalit), die gegebenenfalls auf $Al_2O_3$ geträgert sein können, und amorphe, z.B. solche aus $SiO_2$ in Betracht. Kohlenstoff-Molekularsieb-Membranen lassen sich durch Carbonisierung von organischen Polymeren, z.B. Polyimid herstellen.

Polymer-Membranen sind "Composit"-Membranen aus einer dichten wasserstoffselektiven Polymerschicht auf einem anorganischen Träger.

Als Membranen auf Basis von Metallen der 4. und/oder 5. Nebengruppe, die mit Palladium beschichtet sind, kommen beispielsweise solche in Betracht, bei denen auf einem nichtporösen Träger auf Basis von Nichtedelmetall, bevorzugt Vanadium, Niob und/ oder Tantel, ein oder bevorzugt zwei Schichten aus Palladium oder Palladiumlegierungen vorliegen.

In Frage kommen auch Membranen aus TiN, nanokristalline Metallschichten, beispielsweise auf $Al_2O_3$ geträgertes Platin oder Ruthenium oder amorphe Membranen.

Geeignet sind ferner gemischt leitende Membranen, die sowohl elektronische als auch ionische Leitfähigkeit aufweisen.

Bevorzugt sind allerdings Membranen, die auf Palladium oder Palladiumlegierungen basieren. Als Legierungen kommen insbesondere Legierungen von Palladium mit Silber und/oder Kupfer in Betracht. Dabei sind besonders bevorzugt Membran auf Basis einer Legierung enthaltend Palladium und zwischen 23 Gew.-% und 25 Gew.-% Silber, bezogen auf das Gesamtgewicht der Legierung. Die Legierung enthält dabei besonders bevorzugt zwischen 75 Gew.-% und 77 Gew.-% Palladium, bezogen auf das Gesamtgewicht der Legierung. Besonders bevorzugt sind ferner Membranen auf Basis einer Legierung enthaltend Palladium und zwischen 34 Gew.-% und 46 Gew.-% Kupfer, bezogen auf das Gesamtgewicht der Legierung. Die Legierung enthält dabei besonders bevorzugt zwischen 54 Gew.-% und 66

Gew.-% Palladium, bezogen auf das Gesamtgewicht der Legierung. Die Palladium- bzw. die Palladiumlegierung- Membranen können weiter mit Zeltenerdmetalle, wie z.B. Gadolinium dotiert werden. Dabei können die Palladium- bzw. die Palladiumlegierung- Membranen übliche weitere Metalle enthalten in üblichen Mengen, die die Permeabilität und Selektivität für Wasserstoff nicht oder zumindest nicht wesentlich beeinträchtigen. Auch diese bevorzugten Membranen können auf porösen Unterstrukturen vorliegen, die die eigentliche Membran fixieren und stabilisieren. Die poröse Unterstruktur kann beispielsweise auf Keramik, Metall oder einem organischen Polymeren basieren, z.B. $TiO_2$ und/oder $Al_2O_3$. Die Herstellung der bevorzugt dichten Metallmembran (bevorzugt Palladium oder Palladiumlegierung) auf einem porösen Träger ist allgemein bekannt und kann beispielsweise durch galvanisches Aufbringen, Sputtern, CVD (Chemical Vapor Deposition) oder bevorzugt allgemein bekannte stromlose nasschemische Beschichtung erfolgen.

Als Membranen sind zudem solche bevorzugt, die katalytisch aktiv sind, besonders bevorzugt die Reaktion von $H_2$ insbesondere mit $O_2$ katalysieren, besonders bevorzugt die Aminierung von Benzol durch Umsetzung von Benzol mit Ammoniak, katalysieren. Dabei kann die katalytisch aktive Schicht bevorzugt in der Unterstruktur der Membran vorliegen.

Die Membran trennt die Retentatseite (Reaktionsseite) von der Permeatseite, wobei der auf der Retentatseite gebildete Wasserstoff durch die Membran auf die Permeatseite gelangt, wo der Wasserstoff durch Reaktion, bevorzugt Reaktion mit Sauerstoff oder einem sauerstoffhaltigen Strom, wie zum Beispiel Luft, bevorzugt in Gegenwart von Katalysatoren, und/oder Stofftransport, bevorzugt mittels eines Gasstroms ("Sweepgas" oder Spühlgas) entfernt wird. Bevorzugt trennt die Membran die Retentatseite (Reaktionsseite) von der Permeatseite, wobei der auf der Retentatseite gebildete Wasserstoff durch die Membran auf der Permeatseite gelangt, wo der Wasserstoff durch chemische Reaktion, die durch einen Wasserstoffoxidationskatalysator katalysiert wird, bevorzugt durch Oxidation mit Sauerstoff, entfernt wird. Die selektive Entfernung des Wasserstoffs an der Permeatseite ermöglicht eine deutliche weitere Absenkung des Wasserstoffpartialdruckes an der Retentatseite der Membran (= Reaktionsseite) und ermöglicht so, die gewünschten hohen Benzolumsätze (> 5 mol % > 10 mol % > 20 mol % bezogen auf die zugegebene Menge Benzol) bei höher Anilinselektivität (> 95 %, > 98 %, > 99 %, Anilinselektivität: mol Anilin/ Summe aller gebildeten Produkte in mol (=Benzolumsatz)).

Eine beispielhafte Anordnung einer Membran (1) auf einem porösen Träger (2), die eine Retentatseite (3), in den Benzol und Ammoniak eingespeist und bevorzugt in Gegenwart von Katalysator (4) umgesetzt werden, von der Permeatseite (5) trennt, ist in der Figur 1 dargestellt. Dabei kann die Membran (1) auf der Retentatseite (siehe rechte Seite der Abbildung) und/oder der Permeatseite des Trägermaterials (2) (siehe linke Seite der Figur) angeordnet sein. In der Figur 1 ist schematisch in der linken Permeatseite die Umsetzung des Wasserstoffs mit Sauerstoff, bevorzugt in Gegenwart von Katalysator, dargestellt, während in der rechten Hälfte der Abtransport, ggf. zusätzlich mit Umsetzung mit Sauerstoff abgebildet ist.

Das erfindungsgemäße Verfahren, d.h. die Aminierung von Benzol durch Umsetzung von Benzol mit Ammoniak wird in Gegenwart von Verbindungen durchgeführt, die die Aminierung katalysieren.

Als Katalysatoren können die für die direkte Aminierung von Benzol mit Ammoniak zu Anilin bekannten Katalysatoren eingesetzt werden. Derartige Katalysatoren sind in der Patentliteratur vielfältig beschrieben und allgemein bekannt. Da gemäß dem erfindungsgemäßen Verfahren eine Entfernung des Wasserstoffs durch physikalischen Transport und nicht durch chemische Umwandlung im Reaktionssystem erfolgt, können auch Katalysatoren Verwendung finden, die keine gegenüber Wasserstoff reaktive Komponenten aufweisen. Als Katalysatoren kommen beispielsweise übliche Metallkatalysatoren in Frage, z.B. solche auf der Basis von Nickel, Eisen, Kobalt, Kupfer, Edelmetallen oder Legierungen dieser genannten Metalle. Als Edelmetalle (EM) können alle Edelmetalle in Betracht kommen, z.B. Ru, Rh, Pd, Ag, Ir, Pt und Au, wobei die Edelmetalle Ru und Rh bevorzugt nicht alleine eingesetzt werden, sondern in Legierung mit anderen Übergangsmetallen, beispielsweise Co, Cu, Fe und Nickel oder deren Gemische. Solche Legierungen werden auch bevorzugt eingesetzt bei Anwendung der anderen Edelmetallen, zum Beispiel sind geträgerte NiCuEM; CoCuEM; NiCoCuEM, NiMoEM, NiCrEM, NiReEM, CoMoEM, CoCrEM, CoReEM, FeCuEM, FeCoCuEM, FeMoEM, FeReEM Legierungen von Interesse, dabei ist EM ein Edelmetall, insbesondere bevorzugt Ag und/oder Au.

Der Katalysator kann in allgemein üblicher Form, z.B. als Pulver oder als in ein Festbett einsetzbares System eingesetzt werden (wie beispielsweise Stränge, Kugeln, Tabletten, Ringe, wobei die katalytisch aktiven Bestandteile gegebenenfalls auf einem Trägermaterial vorliegen können. Als Trägermaterial kommen z.B. anorganische Oxide, beispielsweise $ZrO_2$, $SiO_2$, $Al_2O_3$, MgO, $TiO_2$, $B_2O_3$, CaO, ZnO, BaO, $ThO_2$, $CeO_2$, $Y_2O_3$ und Mischungen dieser Oxide, z.B. Magnesium-Aluminium-oxid, bevorzugt $TiO_2$, $ZrO_2$, $Al_2O_3$, Magnesium-Aluminium-oxid und $SiO_2$, besonders bevorzugt $ZrO_2$ und Magnesium-Aluminium-oxid in Frage. Unter $ZrO_2$ wird sowohl reines $ZrO_2$ als auch das normale Hf-enthaltende $ZrO_2$ verstanden.

Die in dem erfindungsgemäßen Verfahren bevorzugt eingesetzten Katalysatoren können regeneriert werden, z.B. indem man eine reduktive (beispielsweise $H_2$-Atmosphäre) über den Katalysator leitet oder zuerst eine oxidative und anschließend eine reduktive Atmosphäre über oder durch das Katalysatorbett führt.

In dem erfindungsgemäßen Aminierungsverfahren wird Benzol eingesetzt, so dass als Produkt Anilin entsteht.

Als Verbindung, durch die die Aminogruppe eingebracht wird, wird Ammoniak eingesetzt. Dies bedeutet, dass

erfindungsgemäß Benzol, mit Ammoniak umgesetzt wird. Gegebenenfalls können auch Verbindungen Verwendung finden, die unter den Reaktionsbedingungen Ammoniak abspalten.

Die Reaktionsbedingungen in den erfindungsgemäßen Aminierungsverfahren sind unter anderem von dem eingesetzten Katalysator abhängig.

Die Aminierung von Benzol, d.h. die Umsetzung von Benzol mit Ammoniak, erfolgt im Allgemeinen bei Temperaturen von 200 bis 800°C, bevorzugt 300 bis 700°C, besonders bevorzugt 325 bis 600°C und ganz besonders bevorzugt 350 bis 500°C.

Der Reaktionsdruck beträgt bei der Aminierung von Benzol, d.h. die Umsetzung von Benzol mit Ammoniak, bevorzugt 1 bis 900 bar, besonders bevorzugt 1 bis 300 bar, insbesondere 5 bis 125 bar, insbesondere bevorzugt 15 bis 110 bar.

Die Verweilzeit beträgt bei der Aminierung von Benzol im Allgemeinen 15 Minuten bis 8 Stunden, bevorzugt 15 Minuten bis 4 Stunden, besonders bevorzugt 15 Minuten bis 1 Stunde, bei der Durchführung in einem diskontinuierlichen Verfahren. Bei Durchführung in einem bevorzugten kontinuierlichen Verfahren beträgt die Verweilzeit im Allgemeinen 0,1 Sekunden bis 20 Minuten, bevorzugt 0,5 Sekunden bis 10 Minuten. Für die bevorzugten kontinuierlichen Verfahren bedeutet "Verweilzeit" in diesem Zusammenhang die Verweilzeit am Katalysator, für Festbettkatalysator somit die Verweilzeit im Katalysatorbett, für Wirbelschichtreaktoren wird der Syntheseteil des Reaktors (Teil des Reaktors, wo der Katalysator lokalisiert ist) betrachtet.

Die relative Menge des eingesetzten Benzols und des Ammoniaks ist abhängig von den Reaktionsbedingungen. Im Allgemeinen werden mindestens stöchiometrische Mengen des Benzols und des Ammoniaks eingesetzt. Üblicherweise ist es jedoch bevorzugt, einen der Reaktionspartner im stöchiometrischen Überschuss einzusetzen, um eine Gleichgewichtsverschiebung auf die Seite des gewünschten Produkts und somit einen höheren Umsatz zu erreichen. Bevorzugt wird der Ammoniak im stöchiometrischen Überschuss eingesetzt.

Das erfindungsgemäße Aminierungsverfahren kann kontinuierlich, diskontinuierlich oder semi-kontinuierlich durchgeführt werden. Geeignete Reaktoren sind somit sowohl Rührkessel-Reaktoren als auch Rohr-Reaktoren. Typische Reaktoren sind beispielsweise Hochdruck-Rührkessel-Reaktoren, Autoklaven, Festbettreaktoren, Wirbelschichtreaktoren, Wanderbetten, zirkulierende Wirbelschichten, Salzbadreaktoren, Plattenwärmetauscher als Reaktoren, Hordenreaktoren mit mehreren Horden mit/oder ohne Wärmetausch bzw. Abzug/Zufuhr von Teilströmen zwischen den Horden, in möglichen Ausführungen als Radialstrom- oder Axialstromreaktoren, kontinuierlich gerührte Kessel, Blasenreaktoren usw., wobei jeweils der für die gewünschten Reaktionsbedingungen (wie Temperatur, Druck und Verweilzeit) geeignete Reaktor eingesetzt wird. Die Reaktoren können jeweils als einzelner Reaktor (single reactor), als Serie von einzelnen Reaktoren und/oder in Form von zwei oder mehr parallelen Reaktoren eingesetzt werden. Die Reaktoren können in einer AB Fahrweise betrieben werden (alterierende Fahrweise). Das erfindungsgemäße Verfahren kann als Batch-Reaktion, semi-kontinuierliche Reaktion oder kontinuierliche Reaktion durchgeführt werden. Der spezielle Reaktoraufbau und die Durchführung der Reaktion können in Abhängigkeit von dem durchzuführenden Aminierungsverfahren, dem Aggregatzustand des Benzols, den erforderlichen Reaktionszeiten und der Natur des eingesetzten stickstoffhaltigen Katalysators variieren. Bevorzugt wird das erfindungsgemäße Verfahren zur Direktaminierung in einem Hochdruck-Rührkessel-Reaktor, Festbettreaktor oder Wirbelschichtreaktor durchgeführt.

In einer besonders bevorzugten Ausführungsform wird bei der Aminierung von Benzol zu Anilin Festbett- oder Wirbelschicht-Reaktor eingesetzt, wobei eine interne Anordnung der Membrane erfolgt und somit im Syntheseteil Wasserstoff entfernt wird. Ein weiterer Vorteil der Membran, welche mittels eines Spülstroms durchströmt werden kann, ist eine gute Wärmekontrolle des Reaktors: Reaktionswärme kann zugegeben oder bevorzugt abgeführt werden durch heizen oder abkühlen des Spülstroms.

Benzol und Ammoniak können in gasförmiger oder flüssiger Form in die Reaktionszone des jeweiligen Reaktors gegeben werden. Die bevorzugte Phase ist jeweils abhängig von der durchgeführten Aminierung sowie dem eingesetzten Reaktor. Bei der Herstellung von Anilin aus Benzol liegen Benzol und Ammoniak bevorzugt als gasförmige Reaktanden in der Reaktionszone vor. Typischerweise wird Benzol dabei als Flüssigkeit zugeführt; die aufgeheizt wird und verdampft, wobei ein Gas gebildet wird, während Ammoniak entweder in gasförmiger Form oder in überkritischer Phase in der Reaktionszone vorliegt. Es ist ebenfalls möglich, dass Benzol zumindest zusammen mit Ammoniak in überkritischer Phase vorliegt.

Benzol und Ammoniak können gemeinsam in die Reaktionszone des Reaktors gegeben werden, zum Beispiel als vorgemischter Reaktandenstrom, oder getrennt. Bei einer getrennten Zugabe können der Kohlenwasserstoff und die Aminkomponente entweder gleichzeitig, zeitversetzt oder nacheinander in die Reaktionszone des Reaktors gegeben werden. Bevorzugt erfolgen die Zugabe des Ammoniaks und die Zugabe des Benzols zeitversetzt.

Gegebenenfalls werden in dem erfindungsgemäßen Verfahren weitere Coreaktanden, Cokatalysatoren oder weitere Reagenzien in die Reaktionszone des Reaktors gegeben, jeweils in Abhängigkeit von der durchgeführten Aminierung. Zum Beispiel kann bei der Aminierung von Benzol Sauerstoff oder ein Sauerstoff enthaltendes Gas in die Reaktionszone des Reaktors gegeben werden, als Coreaktand. Die relative Menge des gasförmigen Sauerstoffs, der in die Reaktionszone gegeben werden kann, ist variabel und unter anderem von dem eingesetzten Katalysatorsystem

abhängig. Das molare Verhältnis von gasförmigem Sauerstoff zu Anilin kann zum Beispiel im Bereich von 0,05 zu 1 bis 1 zu 1, bevorzugt 0,1 zu 1 bis 0,5 zu 1 liegen. Es ist aber auch möglich, die Aminierung von Benzol ohne Zugabe von Sauerstoff oder einem Sauerstoff enthaltenden Gas in die Reaktionszone durchzuführen. Bevorzugt wird das sauerstoffenthaltende Gas an der Permeatseite des Membrans zugegeben und dort katalytisch Wasserstoff zu Wasser oxidiert. Dabei wird die Wasserstoffkonzentration an der Permeatseite bevorzugt möglichst nahe null gehalten.

Im Anschluss an die Aminierung kann die Isolierung des gewünschten Produktes nach dem Fachmann bekannten Verfahren erfolgen.

Ein bevorzugtes Verfahrensschema ist in der Figur 2 skizziert. Dabei haben die Hinweiszeichen die folgenden Bedeutungen:

1: Zuführung Benzol
2: Zuführung Ammoniak
3: Abtrennung von Anilin beispielsweise nach Teilkondensation
4: Membran
5: Katalysator
6: Entfernung von Wasserstoff durch chemische Umwandlung und/oder Abtransport

Das erfindungsgemäße Verfahren zur Herstellung von Anilin durch Umsetzung von Benzol mit Ammoniak erfolgt somit derart, dass man ein Reaktionsgemisch, in das Benzol und Ammoniak getrennt oder gemeinsam bevorzugt kontinuierlich eingespeist werden, in einem Kreislauf führt, der einen Membranreaktor mit Katalysator und Wasserstoffpermeabler Membran aufweist, der Druck des Reaktionsgemisches enthaltend Benzol und Ammoniak in Gegenwart von Katalysator im Membranreaktor zwischen 10 und 150 bar beträgt, und man kontinuierlich Anilin bevorzugt nach Kondensation des Anilins aus dem Kreislauf entfernt. Dabei wird der durch die Membran diffundierte Wasserstoff auf der Permeatseite des Membranreaktors, d.h. auf der vom Reaktionsgemisch abgewandten Seite der Membran, bevorzugt abtransportiert, z.B. mittels Unterdruck oder bevorzugt mittels eine Gasstroms, z.B. Luft oder Stickstoff, und/oder durch chemische Umwandlung, bevorzugt durch Reaktion mit Sauerstoff oder Luft, entfernt. Dabei wird der Transmembrandruck bevorzugt hoch ($\Delta P$ bevorzugt zwischen 1 und 100 bar) eingestellt. Der einstellbare Transmembrandruck wird dabei jedoch limitiert durch die mechanische Stabilität der Membran, dadurch ergibt sich ein Transmembrandruck für Pd oder Palladiumlegierung auf Keramik "Composit" Membranen von typischerweise zwischen 5 und 50 bar.

## Patentansprüche

1. Verfahren zur Herstellung von Anilin, **dadurch gekennzeichnet, dass** man ein Reaktionsgemisch, in das Benzol und Ammoniak getrennt oder gemeinsam eingespeist werden, in einem Kreislauf führt, der einen Membranreaktor mit Katalysator und integrierter Wasserstoffabtrennung durch eine Wasserstoffpermeable Membran aufweist, dass man Wasserstoff durch die Wasserstoffpermeable Membran aus dem Reaktionsgemisch entfernt, wobei die Membran die Retentatseite (Reaktionsseite) von der Permeatseite trennt und der auf der Retentatseite gebildete Wasserstoff durch die Membran auf die Permeatseite gelangt, wo der Wasserstoff durch Reaktion und/oder Stofftransport entfernt wird, und der Druck des Reaktionsgemisches enthaltend Benzol und Ammoniak in Gegenwart von Katalysator im Membranreaktor zwischen 10 und 150 bar beträgt, und man Anilin aus dem Kreislauf entfernt.

2. Verfahren nach Anspruch 1, **dadurch gekennzeichnet, dass** die Membran eine Permeanz für Wasserstoff von größer 10 $m^3 / (m^2 \times h \times bar^{0,5})$ aufweist.

3. Verfahren nach Anspruch 1, **dadurch gekennzeichnet, dass** die Membran auf Palladium basiert.

4. Verfahren nach Anspruch 1, **dadurch gekennzeichnet, dass** die Membran auf Palladiumlegierungen basiert.

5. Verfahren nach Anspruch 4, **dadurch gekennzeichnet, dass** die Membran auf einer Legierung enthaltend Palladium und zwischen 23 Gew.-% und 25 Gew.-% Silber, bezogen auf das Gesamtgewicht der Legierung, basiert.

6. Verfahren nach Anspruch 4, **dadurch gekennzeichnet, dass** die Membran auf einer Legierung enthaltend Palladium und zwischen 34 Gew.-% und 46 Gew.-% Kupfer, bezogen auf das Gesamtgewicht der Legierung, basiert.

7. Verfahren nach einem der Ansprüche 1 bis 6, **dadurch gekennzeichnet, dass** die Membran eine Dicke zwischen 0,1 $\mu$m und 25 $\mu$m aufweist.

8. Verfahren nach einem der Ansprüche 1 bis 7, **dadurch gekennzeichnet, dass** die Membran auf einer porösen keramischen und/oder metallischen Stützschicht fixiert ist.

9. Verfahren nach Anspruch 1, **dadurch gekennzeichnet, dass** die Membran die Retentatseite (Reaktionsseite) von der Permeatseite trennt, der auf der Retentatseite gebildete Wasserstoff durch die Membran auf die Permeatseite gelangt, wo der Wasserstoff durch chemische Reaktion, die durch einen Wasserstoffoxidationskatalysator katalysiert wird, entfernt wird.

10. Verfahren nach Anspruch 1, **dadurch gekennzeichnet, dass** man die Herstellung von Anilin bei Temperaturen zwischen 200 und 800°C durchführt.

## Claims

1. A process for preparing aniline, which comprises conducting a reaction mixture into which benzene and ammonia are fed separately or together in a circulation system which has a membrane reactor with catalyst and integrated hydrogen removal by means of a hydrogen-permeable membrane, removing hydrogen from the reaction mixture by means of the hydrogen-permeable membrane, the membrane separating the retentate side (reaction side) from the permeate side and the hydrogen formed on the retentate side passing through the membrane to the permeate side, where the hydrogen is removed by reaction and/or mass transfer, and the pressure of the reaction mixture comprising benzene and ammonia in the presence of catalyst in the membrane reactor being between 10 and 150 bar, and removing aniline from the circulation system.

2. The process according to claim 1, wherein the membrane has a permeance for hydrogen of greater than 10 $m^3$ / ($m^2$ x h x $bar^{0.5}$).

3. The process according to claim 1, wherein the membrane is based on palladium.

4. The process according to claim 1, wherein the membrane is based on palladium alloys.

5. The process according to claim 4, wherein the membrane is based on an alloy comprising palladium and between 23% by weight and 25% by weight of silver based on the total weight of the alloy.

6. The process according to claim 4, wherein the membrane is based on an alloy comprising palladium and between 34% by weight and 46% by weight of copper based on the total weight of the alloy.

7. The process according to any of claims 1 to 6, wherein the membrane has a thickness between 0.1 $\mu$m and 25 $\mu$m.

8. The process according to any of claims 1 to 7, wherein the membrane is fixed on a porous ceramic and/or metallic supporting layer.

9. The process according to claim 1, wherein the membrane separates the retentate side (reaction side) from the permeate side, the hydrogen formed on the retentate side passes through the membrane to the permeate side, where the hydrogen is removed by chemical reaction which is catalyzed by a hydrogen oxidation catalyst.

10. The process according to claim 1, wherein the preparation of aniline is carried out at temperatures between 200 and 800°C.

## Revendications

1. Procédé pour la production d'aniline, **caractérisé en ce qu'**on conduit un mélange réactionnel, dans lequel du benzène et de l'ammoniac sont introduits séparément ou ensemble, dans un circuit qui comporte un réacteur à membrane avec catalyseur et séparation d'hydrogène intégrée, au moyen d'une membrane perméable à l'hydrogène, **en ce qu'**on sépare l'hydrogène du mélange réactionnel au moyen de la membrane perméable à l'hydrogène, la membrane séparant le côté rétentat (côté réaction) du côté perméat et l'hydrogène formé du côté rétentat passant à travers la membrane du côté perméat, où l'hydrogène est évacué par réaction et/ou transport de substance, et la pression du mélange réactionnel contenant du benzène et de l'ammoniac en présence de catalyseur dans le

réacteur à membrane est comprise entre 10 et 150 bars, et on évacue l'aniline du circuit.

2. Procédé selon la revendication 1, **caractérisé en ce que** la membrane présente une perméabilité pour l'hydrogène de plus de 10 $m^3/m^2 \times h \times bar^{0,5}$).

3. Procédé selon la revendication 1, **caractérisé en ce que** la membrane est à base de palladium.

4. Procédé selon la revendication 1, **caractérisé en ce que** la membrane est à base d'alliages de palladium.

5. Procédé selon la revendication 4, **caractérisé en ce que** la membrane est à base d'un alliage contenant du palladium et entre 23 % en poids et 25 % en poids d'argent, par rapport au poids total de l'alliage.

6. Procédé selon la revendication 4, **caractérisé en ce que** la membrane est à base d'un alliage contenant du palladium et entre 34 % en poids et 46 % en poids de cuivre, par rapport au poids total de l'alliage.

7. Procédé selon l'une quelconque des revendications 1 à 6, **caractérisé en ce que** la membrane présente une épaisseur comprise entre 0,1 µm et 25 µm.

8. Procédé selon l'une quelconque des revendications 1 à 7, **caractérisé en ce que** la membrane est fixée sur une couche de support métallique et/ou céramique poreuse.

9. Procédé selon la revendication 1, **caractérisé en ce que** la membrane sépare le côté rétentat (côté réaction) du côté perméat, l'hydrogène formé du côté rétentat passe à travers la membrane du côté perméat, où l'hydrogène est évacué par une réaction chimique qui est catalysée par un catalyseur d'oxydation d'hydrogène.

10. Procédé selon la revendication 1, **caractérisé en ce qu'**on effectue la production d'aniline à des températures comprises entre 200 et 800 °C.

FIG.1

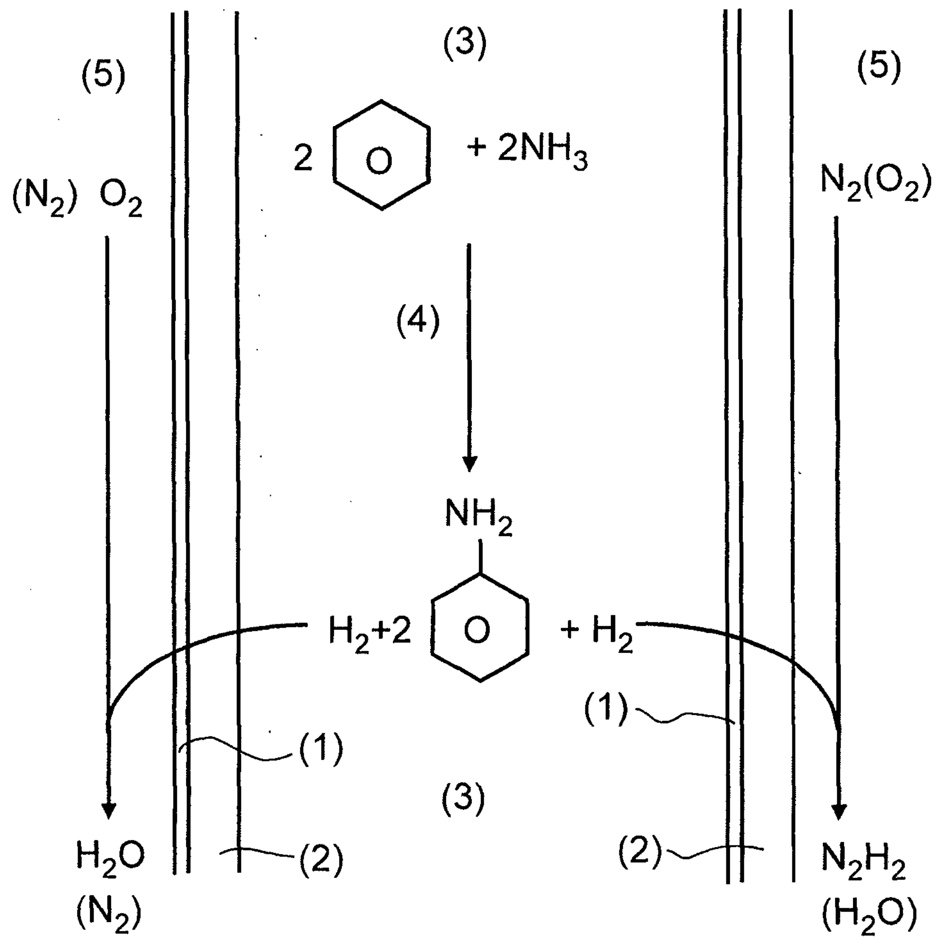

FIG.2

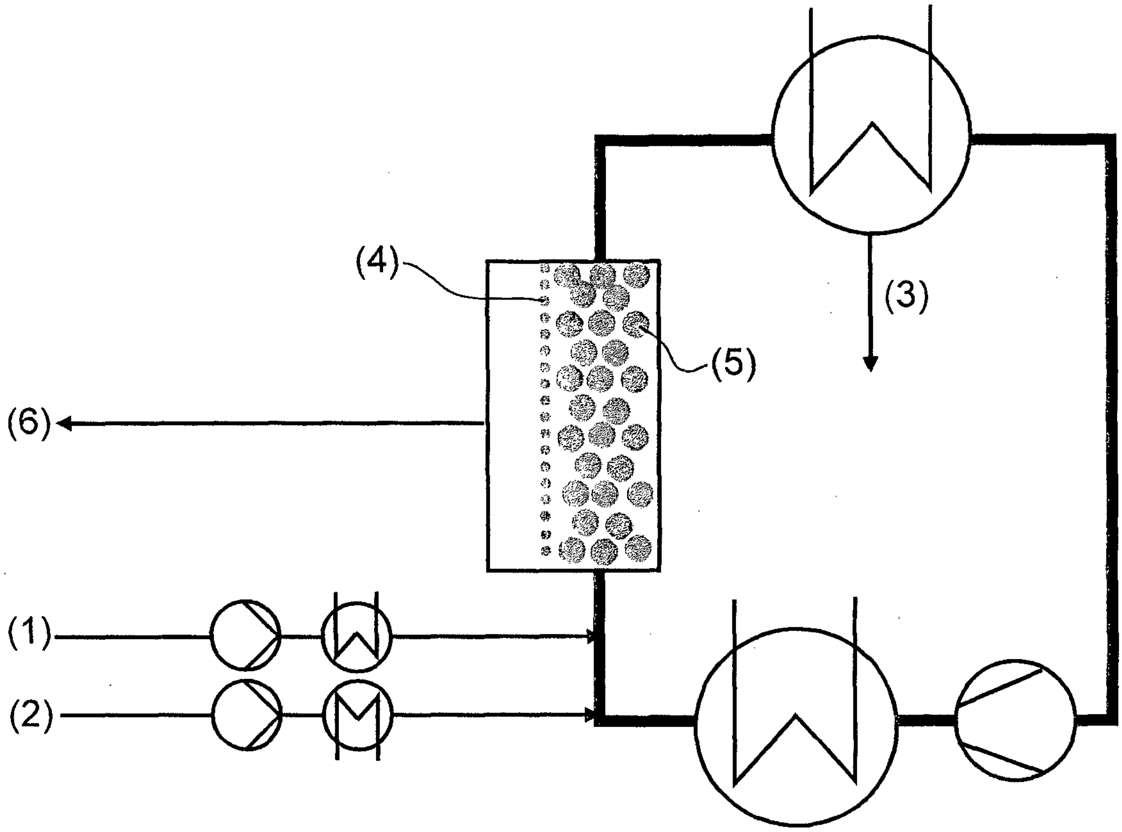

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- CN 1555921 A
- CA 553988
- US 3919155 A
- US 3929889 A
- US 4001260 A
- US 4031106 A
- DE 19634110
- WO 0009473 A
- WO 9910311 A
- WO 0069804 A
- WO 0032512 A


### In der Beschreibung aufgeführte Nicht-Patentliteratur


- **WIBAUT.** *Berichte,* 1917, vol. 50, 541-546